(19) Europäisches Patentamt
European Patent Office
Office européen des brevets

(11) **EP 2 170 262 B1**

(12) **EUROPEAN PATENT SPECIFICATION**

(45) Date of publication and mention
of the grant of the patent:
**21.08.2013 Bulletin 2013/34**

(51) Int Cl.:
*A61K 8/65* (2006.01)     *A61K 8/97* (2006.01)
*A61K 8/98* (2006.01)     *A61Q 19/08* (2006.01)

(21) Application number: **08790920.6**

(22) Date of filing: **01.07.2008**

(86) International application number:
**PCT/JP2008/062252**

(87) International publication number:
**WO 2009/008397 (15.01.2009 Gazette 2009/03)**

(54) **COSMETIC COMPOSITION**

KOSMETISCHE ZUSAMMENSETZUNG

COMPOSITION COSMÉTIQUE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **06.07.2007 JP 2007178574**

(43) Date of publication of application:
**07.04.2010 Bulletin 2010/14**

(73) Proprietor: **FUJIFILM Corporation
Tokyo 106-8620 (JP)**

(72) Inventors:
• **TASHIRO, Tomoko
Ashigarakami-gun
Kanagawa 258-8577 (JP)**
• **KUBO, Toshiaki
Ashigarakami-gun
Kanagawa 258-8577 (JP)**
• **KAWABUCHI, Tatsuo
Tokyo 106-8620 (JP)**
• **SUDO, Yukio
Ashigarakami-gun
Kanagawa 258-8577 (JP)**

(74) Representative: **Morpeth, Fraser Forrest
Fujifilm Imaging Colorants Limited
Intellectual Property Group
P.O. Box 42
Hexagon Tower
Blackley, Manchester M9 8ZS (GB)**

(56) References cited:
EP-A- 1 616 556     JP-A- 2 049 091
JP-A- 5 155 736     JP-A- 2004 244 369
JP-A- 2005 047 860     JP-A- 2006 151 847
JP-A- 2006 342 107     US-A1- 2007 092 462

**Description**

TECHNICAL FIELD

The present invention relates to a cosmetic composition, and more particularly to a cosmetic composition containing a carotenoid.

BACKGROUND ART

Mentioned as typical changes in the human skin due to aging are wrinkles and sagging, which are serious concerns about the skin for advanced age group or women. However, at present, effective treatments for wrinkles and sagging of the skin are hardly found. Thus, various studies on aging have been increasingly conducted in view of the coming aging society.

At present, the wrinkles and sagging of the human skin are considered to be caused by reduction in skin elasticity, and influences of aging, dryness, oxidation, sunlight (ultraviolet rays), etc., are involved. Mentioned as specific histological phenomenons are changes in extracellular matrix components, such as collagen, elastin, and glycosaminoglycan in dermis. A qualitative and/or quantitative reduction in the extracellular matrix components are regarded as major factors of skin aging.

Carotenoids are yellow to red terpenoid pigments which naturally occur, and can be found in plants, algae, and bacteria. Astaxanthins (including astaxanthin, esters thereof, and the like) which are a kind of carotenoids are widely distributed in animals and plants in nature, and are mainly used as a reviver for farmed fishes or farm-raised chickens. Astaxanthin is known to have functions such as an antioxidant effect, an anti-inflammatory effect (e.g., Japanese Patent Application Laid-Open (JP-A) No. 2-49091), an anti-skin aging effect (e.g., JP-A No. 5-155736), an effect of preventing the formation of spots or wrinkles (e.g., JP-A No. 2005-47860). Therefore; the addition of astaxanthin to cosmetic materials and the like have been examined and carried out.

Moreover, it has been already known that a collagen peptide is useful to improve functions of a cosmetic composition. For example, there has been disclosed a technique of decomposing a collagen component by a cysteine protease to thereby obtain a peptide composition, which is useful for cosmetic materials (e.g., JP-A No. 2004-244369). There has also been disclosed that a collagen peptide composition having a specific molecular weight distribution is excellent in various functions as a cosmetic material (a tyrosinase inhibitory activity, an SOD-like activity, a collagen synthesis promotion activity, etc.) (e.g., JP-A Nos. 2006-151847 and 2006-342107).

DISCLOSURE OF INVENTION

The development of a cosmetic composition which exhibits an anti-skin aging effect without impairing the effects of carotenoid has been desired.

The present invention aims to provide a cosmetic composition having an anti-skin aging effect.

The present invention provides the following cosmetic compositions.

<1> A cosmetic composition, containing a carotenoid (A), a collagen (B) having a weight average molecular weight of 40,000 or more, and a collagen peptide (C) having a weight average molecular weight of from 200 to 5,000.

<2> The cosmetic composition according to item <1>, wherein the carotenoid (A) is at least one selected from the group consisting of astaxanthin and derivatives thereof.

<3> The cosmetic composition according to item <1> or <2>, further containing at least one selected from the group consisting of ascorbic acid and derivatives thereof, and tocopherol and derivatives thereof.

<4> The cosmetic composition according to item <3>, wherein the at least one selected from the group consisting of ascorbic acid and derivatives thereof, and tocopherol and derivatives thereof includes at least one selected from the group consisting of magnesium ascorbyl phosphorate, sodium ascorbyl phosphate, ascorbyl-2-glucoside, and sodium ascorbate.

<5> The cosmetic composition according to item <3>, wherein the at least one selected from the group consisting of ascorbic acid and derivatives thereof, and tocopherol and derivatives thereof includes at least one selected from the group consisting of tocopherol and tocotrienol.

<6> The cosmetic composition according to any one of items <1> to <5>, wherein the collagen peptide (C) includes an oligopeptide having 2 to 6 peptide bonds.

<7> The cosmetic composition according to any one of items <1> to <6>, further containing an amino acid or a derivative thereof.

<8> The cosmetic composition according to any one of items <1> to <7>, wherein the collagen peptide (C) is derived from a fish.

<9> The cosmetic composition according to any one of items <1> to <8>, wherein the collagen (B) is derived from a fish.

<10> The cosmetic composition according to any one of items <1> to <9>, wherein the carotenoid (A) is contained in emulsion particles in an oil-in-water water dispersion.

<11> The cosmetic composition according to item <10>, wherein the average particle diameter of the emulsion particles is 200 nm or less.

The present invention can provide a cosmetic composition having an anti-skin aging effect.

BEST MODE FOR CARRYING OUT THE INVENTION

<Cosmetic composition>

The cosmetic composition of the present invention has a feature of containing a carotenoid (A), a collagen (B) having an average molecular weight of 40,000 or more, and a collagen peptide (C) having an average molecular weight of from 200 to 5,000.

This feature makes it possible to obtain a cosmetic composition having an outstanding anti-skin aging effect.

The "anti-skin aging" as used herein refers to the prevention of changes in the skin tissues caused by aging, UV irradiation, etc. For example, improvement of skin resiliency, reduction of skin dullness, and maintenance and improvement of moist feeling and skin elasticity can be mentioned. Moreover, the "average molecular weight" as used herein should be understood as "weight average molecular weight" unless otherwise specified.

The cosmetic composition of the present invention may be any one of an aqueous composition, an oily composition, and an emulsion composition.

Examples of the aqueous composition include lotion, serum, and an aqueous gell. Examples of the oily composition include cleansing oil and an oily gell. Examples of the emulsion composition include cream, milky lotion, and sun screen.

As a form of the emulsion, besides an O/W emulsion and a W/O emulsion, a multiphase emulsion (W/O/W, O/W/O) and the like are mentioned.

A carotenoid is oil soluble. Thus, when the carotenoid is introduced into an oil phase of an oily composition or an emulsion composition, the carotenoid is in a state of being dissolved in another oil soluble component.

When the carotenoid is introduced into an aqueous composition or an aqueous phase of an emulsion composition, the carotenoid is in a state of being dispersed in water. In this case, a cosmetic composition may be produced by a method which involves preparing a water dispersion in which the carotenoid is emulsified in water beforehand, and then mixing the water dispersion with the aqueous composition or the emulsion composition or may be produced by adding the carotenoid in a process of producing a cosmetic composition. The aqueous composition and the emulsion composition will be described later.

Hereinafter, the carotenoid, the collagen, and the collagen peptide in the present invention will be described.

[Carotenoid (A)]

As the carotenoid in the cosmetic composition of the present invention, any carotenoid derived from plants, algae, and bacteria may be mentioned. Moreover, the carotenoid in the cosmetic composition of the present invention is not limited to carotenoids derived from nature. Any carotenoid obtained by an ordinary method may be mentioned as the carotenoid usable in the present invention.

As the carotenoid, hydrocarbons (carotenes), alcohol derivatives (xanthophylls) thereof, and esters thereof are mentioned. In the present invention, these compounds are included in the scope of "carotenoid" unless otherwise specified.

Examples of the carotenoid include actinioerythrol, astaxanthin, bixin, canthaxanthin, capsanthin, capsorubin,β-8'-apo-carotenal (apo-carotenal), β-12'-apo-carotenal, α-carotene, β-carotene, "carotene" (a mixture of α-carotene and β-carotene), γ-carotene, δ-carotene, β-cryptoxanthin, echinenone, palm oil carotene, lutein, lycopene, bioleryThrine, zeaxan-

thin, and esters of a substance containing a hydroxyl or carboxyl group among the above.

These carotenoids exist in nature generally in the forms of a *cis*-isomer and a *trans*-isomer. In the case of a synthetic substance, they may exist also in the form of a racemic mixture.

In general, carotenoids can be extracted from plant materials. These carotenoids have various functions. For example, lutein extracted from the petal of marigold is widely used as a raw material for feed for domestic fowls, and has a function of coloring the skin and fat of domestic fowls, and eggs of domestic fowls.

It is preferable that the carotenoid used in the present invention be oily at room temperatiue from the viewpoints of, for example, ease of handling and dispersing emulsification. Mentioned as a particularly preferable example is an astaxanihin having, for example, an antioxidant effect, an anti-inflammatory effect, an anti-skin aging effect, and a whitening effect, and known as a yellow to red coloring agent.

As such an astaxanthin, an astaxanthin which has been extracted from a natural material using supercritical carbon dioxide gas is more preferable from the view point of odor when formed into a powder.

An astaxanthin is a red pigment having a maximum absorption at 476 nm (in ethanol) and 468 nm (in hexane) and belongs to xanthophylls which are a class of carotenoids (Davies, B.H.: In "Chemistry and Biochemistry of Plant Pigments", T.W.Goodwin ed., 2nd ed., 38-165, Academic Press, NY, 1976.) The chemical structure of astaxanthin is 3,3'-dihydroxy-$\beta,\beta$-carotene-4, 4'-dione ($C_{40}H_{52}O_4$ with a molecular weight of 596.82).

In the present invention, unless otherwise specified, this astaxanthin and derivatives thereof, such as astaxanthin esters, are collectively referred to as "astaxanthin".

Astaxanthin have isomers different in steric configuration of the hydroxy groups at the 3- and 3'-positions of the cyclic structures which exist at both ends of a molecule, including three kinds of isomers - a 3S,3S'-isomer, a 3S, 3R'-isomer (meso-isomer), and a 3R,3R'-isomer. Furthermore, there exist a *cis*-isomer and a *trans*-isomer with respect to the conjugated double bond at the center of a molecule. For example, all *cis*-isomers, 9-*cis* isomer, 13-*cis* isomer, etc., are mentioned.

The hydroxy group at the 3(3')-position can form an ester with a fatty acid. An astaxanthin obtained from krill is a diester having two fatty acids bonded thereto (Yamaguchi, K., Miki, W., Toriu, N., Kondo, Y., Murakami, M., Konosu, S., Satake, M., Fujita, T.: The composition of carotenoid pigments in the antarctic krill Euphausia superba, Bull. Jap. Sos. Sci. Fish., 1983,49, p.1411-1415.). An astaxanthin obtained from H. pluvialis is a 3S,3S'-isomer, and monoesters each having a single fatty acid bonded thereto are contained at high proportions (Renstrom, B., Liaaea-Jensen, S.: Fatty acids of some esterified carotenols, Comp. Biochem. Physiol. B, Comp. Biochem., 1981, 69, p. 625-627).

Moreover, an astaxanthin obtained from Phaffia Rhodozyma is a 3R,3R'-isomer (Andrewes, A.G., Starr, M.P.: (3R, 3'R)-Asttaxanthin from the yeast Phaffa rhodozyma, Phytochem., 1976,15, p.1009-1011), and usually has the structure opposite to the 3S,3S'-isomer which is found in nature. Moreover, an astaxanthin obtained from Phaffia Rhodozyma exists in the form of a free isomer, i.e., it is not in the form of an ester with a fatty acid (Andrewes, A.G., Phaffia, HJ., Starr, M.P.: Carotenids of Phaffia rhodozyma, a red pigmented fermenting yeast, Phytochem., 1976, 15, p. 1003-1007).

Astaxanthin and esters thereof are first isolated from a lobster (Astacus gammarus L.) by R. Kuhn et al., and their estimated structures have been disclosed (Kuhn, R., Soerensen, N.A.: The coloring matters of the lobster (Astacus gammarusL.), Z. Angew. Chem., 1938, 51, p. 465-466). Since then, the following have been revealed: astaxanthin is widely distributed in nature, astaxanthin generally exists as astaxanthin fatty acid esters, and astaxanthin also exists in Crustacea or the like as astaxanthin proteins (ovorubin, crustacyanine) in which astaxanthin is bonded to proteins (Cheesman, D.F.: Ovorubin, a chromoprotein from the eggs of the gastropod mollusc Pomacea canaliculata, Proc. Roy. Soc.B, 1958, 149, p. 571-587).

The astaxanthin or ester thereof contained in the water dispersion or the cosmetic composition of the present invention may be in the form of an astaxanthin-containing oil isolated and extracted from a natural product containing astaxanthin and/or esters thereof. Mentioned as such an astaxanthin-containing oil are, for example, extracts from cultures obtained by culturing red yeast Phaffia, green algae haematococcus, oceanic bacteria, or the like, and extracts from Antarctic krill or the like.

Astaxanthin has two hydroxyl groups in the molecule, and it is known that the hematococcus alga extract (pigment derived from hematococcus alga) is different from the pigment derived from krill in that the former contains monoesters as a main component while the latter contains diesters as a main component

An astaxanthin usable in the present invention may be any of the above-mentioned extracts, or a substance obtained by suitably purifying the extract as necessary, or a synthetic product As the astaxanthin, a substance extracted from hematococcus alga (hereinafter, sometimes referred to as a hematococcus alga extract) is particularly preferable in terms of quality and productivity.

Specifically mentioned as the origins of hematococcus alga extracts usable in the present invention are, for example, *Haematococcus pluvialis, Haematococcus lacustris, Haematococcus capensis, Haematococcus droebakensis,* and *Haematococcus zimbabwiensis.*

There is no limitation on a method of culturing hematococcus alga usable in the present invention, and various methods disclosed in, for example, JP-A No. 8-103288 can be employed insofar a vegetative cell is transformed to a cyst cell, which is a dormant cell.

A hematococcus alga extract usable in the present invention can be obtained by crushing, as required, a cell wall of the above-mentioned raw material and adding an organic solvent, such as acetone, ether, chloroform, and alcohol (ethanol, methanol, etc.) or an extraction solvent, such as carbon dioxide in a supercritical state, so as to carry out extraction according to a method disclosed in, for example, JP-A No. 5-68585.

The hematococcus alga extract contains astaxanthin or esters thereof as a pure pigment, and the content of the esters is generally 50 mol% or higher, preferably 75 mol% or higher, and more preferably 90 mol% or higher, similarly as a pigment described in JP-A No. 2-49091.

Moreover, widely-marketed hematococcus alga extracts may be widely used in the present invention. For example, ASTOTS-S, ASTOTS-2.5O, ASTOTS-50, ASTOTS-10O, etc., which are manufactured by Takeda Shiki Co., Ltd.; ASTAREAR oil 50F, ASTAREAR oil 5F, etc., manufactured by Fuji Chemical Industry Co., Ltd.; BIOASTINSCE7 manufactured by Toyo Koso Kagaku Co., Ltd.; etc., are mentioned.

In the present invention, the content of astaxanthin as a pure pigment in the hematococcus alga extract is preferably from 0.001 to 50 mass%, and more preferably from 0.01 to 25 mass%.

In the cosmetic composition of the present invention, the content of carotenoid (A) is preferably from 0.00001 to 1 mass%, more preferably from 0.00005 to 0.5 mass%, and most preferably from 0.0001 to 0.1 mass% from the viewpoint of functional effects and color.

When the content of carotenoid (A) is 0.00001 mass% or higher, the functional effects of the carotenoid contained may be effectively exhibited, and when the content is 0.0001 mass% or higher, higher effects of carotenoid may be expected. However, carotenoid is colored and has a high extinction coefficient. When the concentration of carotenoid is high, a hand may be colored during use and a portion to which a carotenoid-containing cosmetic composition has been applied may become reddish, which may cause problems with the use of the calotenoid-containing composition as a cosmetic composition. The concentration of carotenoid is preferably 1 mass% or lower, more preferably 0.5 mass% or lower, and most preferably 0.1 mass% or lower from the viewpoint of coloring.

<Collagen (B) having an average molecular weight of 40,000 or more>

The cosmetic composition of the present invention contains a collagen having an average molecular weight of 40,000 or more. The molecular weight distribution is not particularly limited.

Moisture retention can be achieved by the use of such a collagen.

As the collagen (B) usable in the present invention, collagens extracted from mammalian collagen tissues and collagens extracted from collagen tissues of fishes may be used without limitation. In recent years, there is a tendency that use of extracts from mammals in cosmetic compositions is worried about from the viewpoint of safety. Thus, collagens derived from fishes are preferable considering the above. As the raw material of collagens derived from fishes, the skin of tuna (yellowfin tuna) and shark are mentioned, for example. As the raw materials of collagens derived from mammals, a pig and a cow are mentioned, for example.

Collagens can be extracted from these raw materials and purified using common methods. More specifically, such a collagens may be obtained by crushing collagen-containing tissues as a raw material, conducting extraction with acid, an alkali solution, or the like, followed by treatment with various enzymes. As enzymes usable in this process, pepsin, trypsin, proctase, and papain can be mentioned, for example. It is preferable that the enzyme treatment contain multi stages, for example, 2 to 4 stages. The reaction time of the enzyme treatment in each stage is preferably from 7 to 48 hours. Before the enzyme treatment, deliming treatment using hydrochloric acid or the like may be performed so as to remove an inorganic substance. Degreasing treatment using ethanol or the like may also be conducted. The above-mentioned collagen may be a water-soluble collagen obtained by heat extraction with water to acquire water solubility. Moreover, the above-mentioned collagen may be a collagen which has been chemically modified. As such a chemical modification, succination, phtalation, or methyl esterification can be mentioned, for example. Due to the chemical modification, the collagen can be dissolved at a pH of from weakly acidic to neutral, and thus has improved compatibility with other cosmetic components. Moreover, collagen which has been freeze-dried by a common method may be used.

In particular, freeze-dried collagen in the form of particles is preferable from the viewpoint of solubility.

There is no limitation on the collagen usable in the present invention insofar as the average molecular weight is 40,000 or more. The collagen obtained by the above-mentioned method or any of the following commercially-available collagens may be used. Here, the average molecular weight of collagen in the present invention refers to a value measured by gel permeation chromatography (GPC: polystyrene standard).

The average molecular weight of collagen is preferably 50,000 or more, and more preferably 60,000 or more. When the average molecular weight of collagen is less than 60,000, moisture retention may be insufficient.

Specific examples of the collagens derived from fishes include Seagem Collagen, Seagem Collagen HV, and Seagem Collagen AS manufactured by Katakura Chikkarin Co., Ltd.; shark-derived atelocollagen SS and shark-derived atelocollagen SS-V manufactured by Koken Co., Ltd.; marine native collagen manufactured by Arista Life Science Corporation; and collagen S-06, collagen SP-03, etc., manufactured by Nitta Gelatin, Inc..

Specific examples of the collagens derived from pigs include pig-derived atelocollagen, pig-derived atelocollagen SS, pig-derived atelocollagen MS, and pig-derived atelocollagen SS-V manufactured by Koken Co., Ltd.; and collagen P manufactured by Nitta Gelatin, Inc.

In the present invention, the collagen (B) may include either a single collagen or a combination of two or more collagens.

In the cosmetic composition of the present invention, the content of the collagen (B) is preferably from 0.00001 to 20 mass%, more preferably from 0.00005 to 10 mass%, and particularly preferably fom 0.0001 to 2 mass% based on the total mass of the composition.

The content of 0.00001 mass% or more is preferable in terms of moisture retention. However, when the concentration of the collagen (B) is high, filmy feeling may be given. Thus, the concentration is preferably 20 mass% or lower, more preferably 10 mass% or lower, and most preferably 2 mass% or lower.

collagen peptide (C) having an average molecular weight of from 200 to 5000>

The cosmetic composition of the present invention includes collagen peptide (C) having an average molecular weight of from 200 to 5,000 (hereinafter, sometimes referred to as "component (C) ").

There is no limitation on the collagen peptide (C) insofar the collagen peptide has an average molecular weight of from 200 to 5,000 so as to improve permeability to the skin. The molecular weight distribution is not particularly limited.

The average molecular weight is preferably from 500 to 4,500, and more preferably from 1,000 to 4,000. When the average molecular weight of collagen peptide exceeds 4,500, the permeability to the skin may decrease.

The component (C) preferably contains oligopeptide having 2 to 6 peptide bonds from the viewpoint of various effects, such as permeability to the skin, more preferably contains collagen tripeptide having 3 amino acid residues (two peptide bonds).

The component (C) can be manufactured using collagen as its starting material. As the collagen, collagens extracted from mammalian collagen tissues and collagens extracted from collagen tissues of fishes may be used without limitation.

As the raw materials of collagens derived from fishes, the skin of tuna (yellowfin tuna) and shark are mentioned, for example. As the raw materials of collagens derived from mammals, a pig and a cow are mentioned, for example, and a pig is preferable. In recent years, there is a tendency that use of extracts from mammals in cosmetic compositions is worried about from the viewpoint of safety. Thus, collagen derived from fishes is preferable considering the above.

The collagen peptide can be obtained by, for example, subjecting the raw material protein of a fish to enzyme treatment using one or two or more kinds of proteolytic enzymes. Here, as a usable proteolytic enzyme, any of an endo-type enzyme and an exo-type enzyme may be used. The activity thereof is preferably 400 units or more per g of protein. As the proteolytic enzyme, bromelain can be used.

Moreover, collagen is hydrolyzed to yield collagen peptides by performing subcritical water treatment using a collagen obtained from a collagen tissue of a mammal or a fish with a common extraction technique, or using a collagen product. The subcritical water refers to a water showing remarkably different properties from water vapor or liquid water in the usual environment, and has a significantly high hydrolysis function, acting as a strong reaction solvent, and demonstrating strong extraction and purification activity.

In order to effectively obtain the collagen peptides by decomposing the collagen by the subcritical water treatment, it is preferable to adjust the subcritical water treatment conditions as follows: a treatment temperature of from 180 to 220°C, a treatment pressure of from 10 to 24 MPa, and a treatment time of from 30 to 120 minutes. For example, when a water-soluble collagen is used as a collagen component-containing raw material, a preferable condition may be as follows: a treatment temperature adjusted to $200 \pm 10$°C, a treatment time adjusted to $60 \pm 30$ minutes, and a treatment pressure adjusted to $16 \pm 4$ MPa. Thus, collagen peptide can be effectively obtained by the subcritical water treatment.

Furthermore, collagen peptides may be manufactured by allowing a collagenase to act on collagen or gelatin. It is preferable to use, as the collagenase to be used here, a collagenase which is derived from bacteria, actinomycetes, fungus, or the like, such as *Clostridium histolyticum* or *Streptomyces parvulus* and which specifically cleaves an amino-terminal-side of a glycine residue of an amino acid sequence peculiar to coiiagen-(Gly-X-Y)n - wherein, Gly represents a glycine residue, X and Y each represent an amino acid residue and may be the same or different, and n is a positive integer: this sequence is a hereinafter sometimes referred to as a "specific amino acid sequence". This is because the use of such collagenase makes it possible to obtain collagen peptides including a large number of peptides containing the specific amino acid sequence. Moreover, the collagenase to be used herein may be a collagenase obtained as a natural product or a collagenase having the above-mentioned specificity obtained through modification by a protein engineering approach.

There is no limitation on the type of amino acid residue, and usually, amino acid residues of naturally-occurring amino acids can be employed. Specifically, any of the following amino acid residues may be used: an alanine residue, a valine residue, a leucine residue, an isoleucine residue, a proline residue, a hydroxyproline residue, a phenylalanine residue, a tryptophan residue, a methionine residue, a serine residue, a threonine residue, a cysteine residue, a glutamine residue, a glycine residue, an asparagine residue, a tyrosine residue, a lysine residue, an arginine residue, a histidine residue, an aspartic acid residue, and a glutamic acid residue (unless otherwise specified, amino acid residues as described herein refer to L-amino acid residues). Moreover, collagen peptides usable in the present invention can be produced by a usually known method, e.g., a method described in JP-A No. 7-82299. For example, collagen peptides may be produced by using a free collagenase or a collagenase immobilized on an immobilization support, such as chitopearl, in a batch method or a column method or a combination of these methods at a reaction temperature that is adjusted preferably to a temperature of from 40 to 45°C.

There is no limitation on the collagen peptide usable in the present invention insofar as the average molecular weight is 200 to 5,000. The collagen peptide obtained by the above-mentioned method or any of the following commercially-available collagens may be used. Here, the average molecular weight of collagen peptide in the present invention refers to a value measured by gel permeation chromatography (GPC: polystyrene standard), as in the case of collagen.

Specific examples of the collagen peptides derived from fishes include IXOS HDL series produced by Nitta Gelatin, Inc.; and Collagen Tripeptide F produced by JELLICE Co., Ltd. Specific examples of the collagen peptides derived from pigs include Collagen Peptide 800F, Super Collagen Peptide SCP series, and Fermented Collagen Peptide LCP series produced by Nitta Gelatin, Inc.; and Collagen Tripeptide M-30, Collagen Tripeptide M-60, and Collagen Tripeptide M-90 produced by JELLICE Co., Ltd.

In the present invention, the above-mentioned specific collagen peptides may be used singly or in combination of two or more members.

In the cosmetic composition of the present invention, the content of the specific collagen peptide is preferably from 0.00001 to 20 mass%, more preferably from 0.00005 to 10 mass%, and particularly preferably from 0.0001 to 2 mass% based on the total mass of the cosmetic composition.

A content of 0.00001 mass% or higher is preferable in terms of anti-aging effects of collagen tripeptide (for example, a tyrosinase inhibitory activity, an SOD-like action, and collagen synthesis promotion). Moreover, the content of the specific collagen peptide is preferably 20 mass% or lower, more preferably 10 mass% or lower, and most preferably 2 mass% or lower.

There is no limitation on a method of adding the collagen (B) and the collagen peptide (C).

The cosmetic composition of the present invention is produced preferably by forming an oil-in-water type water dispersion of carotenoid beforehand, and mixing the carotenoid with an aqueous composition or an emulsion composition at the time of producing the cosmetic composition. Hereinafter, a water dispersion containing carotenoid will be described.

(Water dispersion)

The water dispersion preferably contains a phospholipid and/or a surfactant for the stability of the emulsion particles containing carotenoid, and may further contain one or more other additives as required. Moreover, the emulsion particles preferably contains an oil-soluble antioxidant.

Hereinafter, the phospholipid and the surfactant usable in the water dispersion will be described.

[Phospholipid contained in a water dispersion]

In the present invention, phospholipid refers to a class of complex lipids, and is an ester containing fatty acid, alcohol, phosphoric acid, and a nitrogen compound, and has at least one phosphoric ester and at least one fatty acid ester. The scope of phospholipid includes glycerophospholipids having glycerin as a basic skeleton and sphingophospholipids having sphingosine as a basic skeleton.

Specific exemples of phospholipid usable in the present invention include phosphatidic acid, bisphosphatidic acid, lecithin (phosphatidylcholine), phosphatidylethanolamine, phosphatidyl methylethanolamine, phosphstidylserine, phosphatidylinositol, phosphatidylglycerol, diphosphatidyl glycerol, and sphingomyelin. Further examples include lecithins containing such phospholipids and derived from the following: plants such as soybean, corn, peanut, rapeseed, and wheat; animals such as egg yolk and a cow; and microorganisms, such as Escherichia coli. Lecithins and hydrogenated lecithin as a mixture of the above-mentioned substances can also be used. There is no limitation on the origins of the above-mentioned phospholipids. A purified phospholipid is preferable.

In the present invention, the scope of glycerophospholipid includes a lysolecithin-a glycerophospholipid having one fatty acid residue in one molecule as a result of enzymolysis.

Such a lysolecithin can be obtained by hydrolysis of lecithin with acid or an alkaline catalyst, or by hydrolysis of lecithin with phospholipase $A_1$ or $A_2$.

Mentioned as such lysolecithins are lysophosphatidic acid, lisophosphatidylglycerol lysophosphatidylinositol, lysophosphatidylethanolamine, lysophosphatidylmethylethanolamine, lysophosphatidylcholine (lysolecithin), and lysophosphatidylserine, for example.

Furthermore, as glycerophospholipid typified by the above-mentioned lecithin, hydrogenated or hydroxylated lecithins can also be used in the present invention. For example, hydrogenated lecithins, enzymatically decomposed lecithins, enzymatically decomposed hydrogenated lecithins, and hydroxylecithins are usable.

The hydrogenation is performed by, for example, reacting lecithin with hydrogen in the presence of a catalyst, whereby an unsaturated bond in the fatty acid part is hydrogenated. The oxidation stability of lecithin is improved by hydrogenation. With respect to the hydroxylation, an unsaturated bond in the fatty acid part is hydroxylated by heating lecithin with a high concentration of hydrogen peroxide and an organic acid such as acetic acid, tartaric acid, or butyric acid. The hydrophilicity of lecithin is improved by the hydroxylation.

As the phospholipid, lecithin is particularly preferable from the viewpoint of emulsion stability.

As commercially-available lecithins, LECION series and LECIMAL EL produced by Riken Vitamin Co., Ltd., can be mentioned, for example.

Products with a purity of lecithin of 60 mass% or higher are industrially used as lecithin. However, in the present invention, products having a purity of lecithin of 80 mass% or higher, which are referred to as "high purity lecithin", are preferable, and products having a purity of lecithin of 90 mass% or higher are more preferable.

The purity of the above-mentioned lecithins is preferably in the range as mentioned above from the viewpoint of the formation of oil droplets having small particle diameters and the stability of functional oil-soluble component (e.g., carotinoide and the like).

The lecithin purity (mass%) is calculated by subtracting the weights of a toluene insoluble matter and an acetone-soluble matter; this calculation utilizes the features that lecithin easily dissolves in toluene but is insoluble in acetone. The high purity lecithin is highly lipophilic as compared with lysolecithin, and thus the compatibility between the lecithin and an oily component is high. Thus, the high purity lecithin is preferable in that it can improve the emulsion stability.

The phospholipid used in the present invention can be used singly or as a mixture of two or more members.

From the viewpoint of the emulsion stability, the content of phospholipid in the water dispersion of the present invention is preferably from 0.001 to 20 mass%, and more preferably 0.001 to 10 mass%, with respect to the water dispersion.

[Surfactant contained in a water dispersion]

There is no limitation on a surfactant usable in the water dispersion of the present invention insofar as the surfactant is a water soluble surfactant which dissolves in an aqueous medium. For example, nonionic surfactants whose HLB is 10 or more, preferably 12 or higher are preferable. When the HLB is lower than 10, the emulsification ability may become insufficient. From the viewpoint of the emulsion stability, the HLB is preferably 16 or lower.

Here, the HLB refers to a balance between hydrophilicity and hydrophobicity generally used in the field of surfactants, and a commonly used calculation formula, e.g., Kawakami formula or the like can be used. In the present invention, Kawakami formula is employed.

$$HLB = 7 + 11.7 \log (Mw/Mo),$$

wherein Mw represents the molecular weight of the hydrophilic group(s) and Mo represents the molecular weight of the hydrophobic group(s).

Moreover, the numerical value of HLB indicated in a catalog or the like may be employed.

Moreover, as is clear from the formula, a surfactant having an arbitrary HLB value can be obtained using the additive property of HLB.

There is no limitation on surfactants usable in the present invention, and nonionic surfactants are preferable. Examples of the nonionic surfactants include glycerin fatty acid esters, organic acid monoglycerides, polyglycerine fatty acid esters, propylene glycol fatty acid esters, polyglycerin condensed ricinoleic-acid esters, sorbitan fatty acid esters, and sucrose fatty acid esters More preferable are polyglycerin fatty acid esters, sorbitan fatty acid esters, and sucrose fatty acid esters. Moreover, the above-mentioned surfactants are not necessarily highly purified products obtained by distillation or the like, and may be reaction mixtures.

The polyglycerin fatty acid ester used in the present invention is an ester of a polyglycerin having an average degree of polymerization of 2 or higher, preferably from 6 to 15, and more preferably from 8 to 10 and a $C_{8-18}$ fatty acid, such as caprylic acid, capric acid, lauric acid, myristic acid, palmitic acid, stearic acid, oleic acid, or linoleic acid. Preferable examples of the polyglycerin fatty acid ester include hexaglycerin monooleate, hexaglycerin monostearate, hexaglycerin monopalmitate, hexaglycerin monomyristate, hexaglycerin monolaurate, decaglycerin monooleate, decaglycerin monostearate, decaglycerin monopalmitate, decaglycerin monomyristate, and decaglycerin monolaurate. These polyglycerin fatty acid esters can be used singly or as a mixture of two or more polyglycerin fatty acid esters. Examples of commercially-available polyglycerin fatty acid esters include products of Nikko Chemicals Co., Ltd., such as NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn 1-M, NIKKOL Hexaglyn 1-SV, NIKKOL Hexaglyn 1-O, NIKKOLHexaglyn 3-S, NIKKOL Hexaglyn 4-B, NIKKOL Hexaglyn 5-S, NIKKOL Hexaglyn 5-O, NIKKOL Hexaglyn PR-15, NIKKOL Decaglyn I-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-O, NIKKOL Decaglyn 10-SV, NIKKOL Decaglyn 10-IS, NIKKOL Decaglyn 10-OV, NIKKOL Decaglyn 10-MAC, and NIKKOL Decaglyn PR-20; products of Mitsubishi Chemical Foods Co., Ltd., such as RYOTO-POLYGLYESTER L-10D, L-7D, M-10D, M-7D, P-8D, S-28D, S-24D, SWA-20D, SWA-15D, SWA-10D, O-50D, O-15D, B-100D, B-70D, and ER-60D; products of Taiyo Kagaku CO., LTD., such as Sun Soft Q-17UL, Sun Soft Q-14S, and Sun Soft A-141C; and products of Riken Vitamin Co., Ltd., such as Poem DO-100 and Poem J-0021.

In the sorbitan fatty acid ester used in the present invention, the fatty acid preferably has 8 or more carbon atoms, and more preferably 12 or more carbon atoms. Mentioned as preferable examples of the sorbitan fatty acid ester are sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, and sorbitan trioleate. The above-mentioned sorbitan fatty acid esters can be used singly or as a mixture of two or more sorbitan fatty acid esters. Mentioned as commercially-available sorbitan fatty acid esters are, for example, products of Nikko Chemicals Co., Ltd., such as NIKKOL SL-10, SP-10V SS-10V, SS-10MV, SS-15V, SS-30V, SI-10RV, SI-15RV, SO-10V, SO-15MV, SO-15V, SO-30V, SO-10R, SO-15R, SO-30R, and SO-15EX; and products of Dai-Ichi Kogyo Seiyaku Co., Ltd., such as SOLGEN 30V, 40V, 50V, 90, and 110.

In the sucrose fatty acid ester used in the present invention, the fatty acid preferably has 12 or more carbon atoms, and more preferably has 12 to 20 carbon atoms. Mentioned as preferable examples of the sucrose fatty acid ester are sucrose dioleate, sucrose distearate, sucrose dipalmitate, sucrose dimyristate, sucrose dilaurate, sucrose monooleate, sucrose monostearate, sucrose monopalmitate, sucrose monomyristate, and sucrose monolaurate. In the present invention, the above-mentioned sucrose fatty acid esters can be used singly or as a mixture of two or more sucrose fatty acid esters. Mentioned as commemially-available sucrose fatty acid esters are, for example, products of Mitsubishi Chemical Foods Co., Ltd., such as RYOTO SUGAR ESTER S-070, S-170, S-270, S-370, S-370F, S-570, S-770, S-970, S-1170, S-1170F, S-1570, S-1670, P-070, P-170, P-1570, P-1670, M-1695, O-170, O-1570, OWA-1570, L-195, L-595, L-1695, LWA-1570, B-370, B-370F, ER-190, ER-290, and POS-135; products of Dai-Ichi Kogyo Seiyaku Co., Ltd, such as DK ester SS, F160, F140, F110, F90, F70, F50, F-A50, F-20W, F-10, F-A10E, and COSMELIKE B-30, S-10, S-50, S-70, S-110, S-160, S-190, SA-10, SA-50, P-10, P-160, M-160, L-10, L-50, L-160, L-150A, L-160A, R-10, R-20, O-10, and O-150.

The addition amount of the surfactant is preferably from 0.1 to 50 mass%, more preferably from 0.5 to 20 mass% and still more preferably from 1 to 15 mass%, with respect to the water dispersion.

By adjusting the addition amount to 0.1 mass% or higher, an emulsion having a smaller particle diameter can be obtained and the stability of the emulsion can be sufficiently secured. By adjusting the addition amount to 50 mass% or lower, the foaming of the emulsion can be adjusted to a suitable level. Thus, such an addition amount is preferable.

[Particle diameter of emulsion particles in water dispersion]

From the viewpoint of transparency, the particle diameter of the emulsion particles in the water dispersion may be 200 nm or less, preferably 150 nm or less, and more preferably 100 nm or less, in terms of a volume average particle diameter (median diameter). In the present specification, the term "average particle diameter" refers to a "volume average particle diameter" unless indicated otherwise.

The particle diameter varies depending on factors, such as the types and the use amounts of additive components, emulsification conditions (shearing force, temperature, and pressure) in a production process, the use amount of additives, an oil phase-aqueous phase ratio, and the use amount of surfactant. However, the particle diameter in the range of the present invention causes no problems in practical use.

The particle diameter can be measured by a commercially-available particle size distribution measuring instrument or the like. Known as a particle size distribution measurement method of an emulsion are, for example, an optical microscope method, a confocal laser scanning microscope method, an electron microscope method, an atomic force microscope method, a static light scattering measurement method, a laser diffraction method, a dynamic light scattering method, a centrifugal sedimentation method, an electrical pulse measurement method, a chromatography method, and an ultrasonic attenuation method. Apparatuses corresponding to the respective principles are marketed.

The dynamic light scattering method is preferable when measuring the particle diameter of an emulsion of the present invention from the viewpoint of the particle diameter range in the present invention and the ease of measurement. Mentioned as commercially-available measurement apparatuses employing the dynamic light scattering method are, for example, Nano truck UPA (Nikkiso Co., Ltd.), a dynamic light scattering particle-size-distribution measurement apparatus LB-550 (Horiba, Ltd.), and a Fiber-Optics Particle Analyzer with Autosampler FPAR-1000 (Otsuka Electronics Co., Ltd.). The measurement temperature may be a temperature commonly used for the measurement of the particle diameter, and a measurement temperature of 20°C is preferable,

The particle diameter in the present invention is a value measured at a measurement temperature of 20°C using the dynamic light scattering particle-size-distribution measurement apparatus.

There is no limitation on the water dispersion containing carotenoid in the present invention. For example, the water dispersion containing carotenoid in the present invention can be obtained by a) dissolving a surfactant (water soluble emulsifier) in an aqueous medium to thereby obtain an aqueous phase, b) mixing and dissolving the above-mentioned carotenoid, tocopherol, and phospholipid, and, as required, one or more other optional oil-soluble components to thereby obtain an oil phase, and c) mixing the aqueous phase and the oil phase under stirring, so as to conduct dispersing emulsification.

The cosmetic composition of the present invention may contain, as required, one or more ingredients selected from water, a polyhydric alcohol, a water soluble polymer compound, an oil soluble component (e.g., oil, wax), an antiseptic agent, an antioxidant, and fragrant material, which are generally used in cosmetic compositions.

(Antioxidant)

It is preferable to add an antioxidant to the cosmetic composition of the present invention so as to maintain the stability of carotenoid. There is no limitation on usable antioxidants, and, for example, a class of compounds based on polyphenol, radical scavengers, tocopherols, and ascorbic acid can be mentioned.

As the antioxidant used in the present invention, a hydrophilic antioxidant and/or an oil-soluble antioxidant can be used singly or in combination of two or more antioxidants.

As the antioxidant, at least one member selected from ascorbic acid, ascorbic acid derivatives, tocopherol, and tocopherol derivatives is preferably contained. The antioxidant may be added to an aqueous phase or to an oil phase of the cosmetic composition. When a water dispersion of carotenoid is prepared beforehand, the carotenoid is preferably dispersed and emulsified together with the antioxidant because antioxidative effects are demonstrated more effectively. When the antioxidant is emulsified in the water dispersion, the antioxidant is preferably an oil-soluble antioxidant, and is preferably tocopherol or a tocopherol derivative (for example, tocotrienol).

There is no limitation on the content of the antioxidant in the cosmetic composition. The weight ratio of the amount of antioxidant to the amount of carotenoid in the cosmetic composition is preferably within the range of from 0.1 to 50,000, and more preferably from 0.2 to 10,000, in consideration of effective prevention of the oxidation of carotenoid. In particular, when the antioxidant and carotenoid are emulsified together in emulsion particles of a water dispersion, the content ratio of antioxidant to carotenoid is preferably within the range of from 0.1 to 5 by weight in view of obtaining effective antioxidative property.

(Ascorbic acid or derivatives thereof)

Mentioned as ascorbic acid or ascorbic acid derivatives are ascorbic acid, sodium ascorbate, potassium ascorbate, calcium ascorbate, L-ascorbic acid phosphate, magnesium ascorbyl phosphate, sodium ascorbyl phosphate, ascorbyl sulfate, disodium ascorbyl sulfate, ascorbyl-2-glucoside, etc. Moreover, erythorbic acid or derivatives thereof, such as erythorbic acid, sodium erythorbate, potassium erythorbate, calcium erythorbate, phosphate erythorbate, sulfate erythorbate, etc., can also be mentioned as the ascorbic acid or derivatives thereof in the present invention.

Among the above, from the viewpoint of prevention of fading of carotenoid and dispersion stability of emulsion particles, magnesium ascorbyl phosphate, sodium ascorbyl phosphate, ascorbyl-2-glucoside, and sodium ascorbate are preferable, and magnesium ascorbyl phosphate and sodium ascorbyl phosphate are particularly preferable. Commercially-available ascorbic acids and derivatives thereof can be suitably used. Mentioned are, for example, L-ascorbic acid (Takeda Chemical Industries, FUSO CHEMICAL Co., Ltd., BASF Japan, Daiichi Seiyaku Co., Ltd, etc.), sodium L-ascorbate (Takeda Chemical Industries, FUSO CHEMICAL Co., Ltd., BASF Japan, Daiichi Seiyaku Co., Ltd, etc.), 2-glucoside ascorbate (Tradename: AA-2G, product of Hayashibara Biochemical Laboratories), magnesium L-ascorbic acid phosphate (Tradename: Ascorbic acid PM "SDK", product of Showa Denko K.K.), Tradename: NIKKOL VC-PMG (Nikko Chemicals Co., Ltd.), and Tradename: SEAMATE (Takeda Chemical Industries).

(Tocopherol or derivatives thereof)

Usable tocopherols are not limited, and are selected from a class of compounds including tocopherol and derivatives thereof.

Mentioned as the class of compounds including tocopherol and derivatives thereof are, for example, tocopherol and derivatives thereof, such as dl-α-tocopherol, dl-β-tocopherol, dl-γ-tocopherol, dl-δ-tocopherol, acetic acid dl-α-tocopherol, nicotinic acid-dl-α-tocopherol, linolic acid dl-α-tocopherol, and succinic-acid dl-α-tocopherol, α-tocotrienol, β-tocotrienol, γ-tocotrienol, and δ-tocotrienol. Among the above, tocopherols (dl-α-tocopherol, dl-β-tocopherol, dl-γ-tocopherol, dl-δ-tocopherol), and tocotrienols (α-tocotrienol, β-tocotrienol, γ-tocotrienol, δ-tocotrienol) are preferable.

The above-mentioned tocopherol and derivatives thereof are used in the form of a mixture in many cases, and may be used in such a form as an extracted tocopherol or a mixed tocopherol.

(Other antioxidants)

Mentioned as the class of compounds including polyphenols as usable antioxidants are, for example, flavonoids (such as catechin, anthocyanin, flavone, isoflavone, flavane, flavanone, and rutin), phenolic acids (such as chlorogenic acid, ellagic acid, gallic acid, and propyl gallate), lignans, curcumines, and coumarins. Moreover, since these compounds are contained at high contents in extracts derived from natural products such as the following, the compounds can be used in the form of extracts.

Examples include licorice extracts, cucumber extracts, Mucuna birdwoodiana extracts, gentian (Gentiana triflora) extracts, Geranium thunbergii extracts, cholesterol and derivatives thereof, hawthorn extracts, peony extracts, ginkgo extracts, Baikai skullcup extracts, carrot extracts, Rugosa rose (Maikai) extracts, sanpenzu (cassia) extracts, torumentila extracts, parsley extracts, Paeonia suffruticosa (Moutan Cortex.) extracts, Japanese quince extracts, Melissa extracts, alnus firma fruit extracts, strawberry geranium extracts, rosemary (mannennrou) extracts, lettuce extracts, tea extracts (colong tea, black tea, green tea, etc.), microorganism fermentation metabolic products and Momordica grosvenorii extracts (terms in the brackets describe other names of plants and names of crude drugs). Among the polyphenols, particularly preferable ones include catechin, rosemary extracts, glucosyl rutin, ellagic acid and gallic acid.

As the antioxidants, general commercially available products may be appropriately used. Examples thereof include ellagic acid (manufactured, for example, by Wako Pure Chemicals Industries Ltd., etc.), rosemary extracts (for example, RM-21A, RM-21 E manufactured by Mitsubishi-Kagaku Foods Corp., etc.), catechins (for example, SUNKATOLW-5, No.1: manufactured by Taiyo Kagaku Co., Ltd., etc.), sodium gallate (for example, SUNKATOL manufactured by Taiyo Kagaku Co., Ltd., etc.), rutin, glucosyl rutin, and enzymatically decomposed rutin (for example, RUTIN K-2, P-10 manufactured by Kiriya Chemical Co, Ltd, and αG Rutin manufactured by Hayashibara Biochemical Laboratories, Inc., etc.).

[Oil-soluble component]

The cosmetic composition of the present invention may contain one or more other additional oil-soluble components which dissolve in an oily medium.

As such other oil-soluble components, additional components which are generally used as UV absorbers, anti-oxidants, anti-inflammatory agents, moisturizers, hair protecting agents, dispersants, solvents, whitening agents, anti-spot agents, cell activators, emollient agents, keratolytic agents, antistatic agents, vitamins, metabolic-syndrome improving agents, hypotensors, and sedatives, may be used. Examples thereof include: oils and fats, such as olive oil, camellia oil, macadamia nuts oil, and castor oil; hydrocarbons, such as liquid paraffin, paraffin, vaseline, ceresin, microcrystalline wax, and squalane; waxes, such as carnauba wax, candelilla wax, jojoba oil, yellow wax, and lanolin; esters, such as isopropyl myristate, 2-octyldodecyl myristate, cetyl 2-ethylhexanoate, and diisostearyl malate; fatty acids, such as palmitic acid, stearic acid, and isostearic acid; higher alcohols, such as cetyl alcohol, stearyl alcohol, isostearyl alcohol, and 2-octyldodecanol; silicone oils, such as methylpolysiloxane and methylphenyl polysiloxane; fatty acid esters of glycerin; high-molecular-weight substances; oil-soluble pigments; and oil-soluble proteins. Further examples include various kinds of oils derived from plants and oils derived from animals, which are mixtures of such oil-soluble substances. Other preferable examples of the additional oil-soluble components used for the present invention include vitamin E (except the above-mentioned tocopherol and derivatives thereof), and coenzymes Q, ω-3 oils and fats (oils and fats containing EPA, DHA, linolenic acid, etc.).

(Polyhydric alcohol)

It is preferable for the cosmetic composition of the present invention to contain a polyhydric alcohol in order to exhibit, for example, a moisturizing function and/or a viscosity control function. Moreover, by the addition of the polyhydric alcohol, the water activity of the cosmetic composition can be lowered to thereby suppress propagation of microorganisms.

As the polyhydric alcohol usable in the present invention, any di- or higher- hydric alcohols can be used without limitation.

Examples of the polyhydric alcohol include glycerin, diglycerin, triglycerin, polyglycerin, 3-methyl-1,3-butanediol, 1,3-butylene glycol, isoprene glycol, polyethylene glycol, 1,2-pentanediol, 1,2-hexanediol, propylene glycol, dipropylene glycol, polypropylene glycol, ethylene glycol, diethylene glycol, pentaerythritol, neopentyl glycol, maltitol, reduced starch syrup, fructose, glucose, sucrose, lactitol, palatinit, erythritol, sorbitol, mannitol, xylitol, xylose, glucose, lactose, mannose, maltose, galactose, fructose, inositol, pentaerythritol, malto triose, sorbitol, sorbitan, trehalose, amylolysis sugar, and amylolysis sugar reduced alcohol. Only a single polyhydric alcohol, which may be selected from the above, may be used, or a mixture of two or more polyhydric alcohols, which may be selected from the above, may be used.

(Water-soluble polymer compound)

As a water-soluble polymer compound, a wide variety of synthetic polymers, natural polymers and semi-synthetic polymers may be used. In particular, saccharides, proteins, and glycoprotein complexes are preferable.

Examples of saccharides include, but are not limited to, monosaccharides, disaccharides, oligosaccharides, polysaccharides, dextrin, starch derivatives, gums, mucopolysaccharides, and celluloses.

Among the above, typical examples include, but are not limited to, agarose, arabinose, amylose, amylopectin, acacia gum, gum arabic, arabinogalactan, alkyl glycoside, alginic acid, sodium alginate, propylene glycol alginate, aldose, inulin, oligosaccharide, ghatti gum, curdlan, carrageenan, galactomannan, galactose, xanthan gum, xylose, xyloglucan, chitin, chitosan, guar gum, cluster dextrin, β-glucan, glucuronic acid, glycogen, glycosaminoglycan, glyceraldehyde, glucosamine, glucose, glucomannan, ketose, chondroitin sulfate, psyllium seed gum, gellan gum, cyclodextrin, sucrose, hydroxyethylcellulose, hydroxypropylcellulose, carboxymethylcellulose, methylcellulose, cellobiose, sorbitol, deoxyribose, dextrin, invert sugar, starch, soybean polysaccharide, sugar alcohol, glycoprotein, tragacanth gum, trehalose, hyaluronic acid, fucose, fructose, pullulan, pectin, heparin, hemicellulose, maltose, mannitol, mannan, lactose, and ribose. Among the above-mentioned saccharides, gums and polysaccharides are preferable from the viewpoint of dispersion stability due to increase in viscosity, and xanthan gum, gum arabic, pullulan, etc., are more preferable from the viewpoint of the stability of carotenoids.

As the proteins, any polymer or oligomer in which amino acid residues are polymerized through peptide bonds may be used. More preferable are proteins which are derived from nature and are water soluble.

Proteins may be classified into simple proteins composed of amino acids and complex proteins containing a constituent other than amino acid, and both of them are usable. Examples of simple proteins include gelatin, casein, fibroin, sericin, keratin, and protamine. Examples of complex proteins include a glycoprotein which is a protein bonded to a carbohydrate, a lipoprotein which is a protein bonded to a lipid, a metalloprotein which is a protein bonded to a metal ion, a nucleoprotein which is a protein bonded to a ribonucleic acid, and a phosphoprotein which is a protein bonded to a phosphate group. In general, proteins are often termed based on their raw materials. For example, animal muscle proteins, milk proteins, egg proteins, rice proteins, wheat proteins (wheat gluten), soybean proteins, yeast proteins, and bacteria proteins may be mentioned.

In an embodiment, a mixture of two or more of such proteins is used.

(Amino acids or derivatives thereof)

It is preferable for the cosmetic composition of the present invention to contain an amino acid or a derivative thereof.

Usable amino acids or derivatives thereof may be selected from those usable as ingredients of cosmetic materials, without particular limitations.

Examples of the amino acids or derivatives thereof include amino acids such as glycine, alanine, valine, leucine, isoleucine, serine, threonine, aspartic acid, glutamic acid, cystine, methionine, lysine, hydroxylysine, arginine, histidine, phenylalanine, tyrosin, tryptophan, proline, hydroxyproline, and acetylhydroxyproline, and derivatives of such amino acids.

As the amino acids or derivatives thereof, hydroxyproline and acetylhydroxyproline are preferable among the above.

As the amino acids or derivatives thereof, those synthesized by conventional methods and those commercially available are both usable.

These amino acids and derivatives thereof may be used singly or in combination of two or more thereof.

The total content of the amino acids and the derivatives thereof is not particularly limited, and is preferably from 0.00001 to 10 mass%, more preferably from 0.0001 to 5 mass%, and still more preferably from 0.0005 to 1 mass%, based on the total mass of the cosmetic composition.

The cosmetic composition of the present invention may further contain fragrant material.

Examples of fragrant materials usable in the present invention include natural fragrant materials derived from animals, plants, and minerals and synthetic fragrant materials, such as rose extract, chamomile extract, green tee fragrant material, lavender oil, geranium oil, jasmine oil, bergamot oil, musk oil, ylang ylang oil, limonene, linalool, β-phenylethyl alcohol, 2,6-nonadienal, citral, cyclopentadecanone, eugenol, rose oxide, indole, phenylacetaldehyde dimethyl acetal, and aurantiol.

In the cosmetic composition of the present invention, the aqueous composition containing the component (A), the component (B), and the component (C) can be produced by, for example, mixing the above-mentioned water dispersion and the aqueous composition as described above. The way to add the component (B) and the component (C) is not limited. The component (B) and the component (C) may be independently added to the water dispersion and/or the aqueous composition or may be added in a final stage of the production of the cosmetic composition. There is no limitation on the components to be contained in the aqueous composition, and may be selected from, as necessary, those mentioned as the components of the aqueous phase of the water dispersion, for example.

Moreover, in the cosmetic composition of the present invention, the emulsion composition which contains the component (A), the component (B), and the component (C) can be produced by, for example, mixing the water dispersion and the emulsion composition as described above. Alternatively, when an emulsion composition containing the component (A) is used, the emulsion composition as it is may be used as a cosmetic composition without using the water dispersion. The way to add the component (B) and the component (C) is not particularly limited. The component (B) and the component (C) in the emulsion composition may be independently added to the water dispersion and/or the emulsion composition or may be added in a final stage of the production of the cosmetic composition.

The emulsion composition can be produced using the method of producing the water dispersion and/or a conventional method. There is no limitation on the components to be contained in the emulsion composition, and may be selected, as necessary, from those mentioned as components of the aqueous phase and the oil phase of the water dispersion, for example.

Further, in the cosmetic composition of the present invention, the oily composition containing the component (A), the component (B), and the component (C) may be produced by a conventional method, using ingredients including these components. There is no limitation on the ingredients other than the above-mentioned components, and may be selected from those mentioned as optional components in the cosmetic composition of the present invention.

EXAMPLES

Hereinafter, the present invention will be described with reference to Examples. The Examples should not be construed as limiting the invention. In the following description, "part" and "%" are based on mass unless otherwise specified.

(1) Production of water dispersion (i)

The respective components shown in Table 1 were dissolved under heating at 70°C for 1 hour to thereby obtain an aqueous composition.
The respective components shown in Table 2 were dissolved under heating at 70°C for 1 hour to thereby obtain an oil phase composition.
Table 1

| Sucrose stearate (HLB = 16) | 13 g |
| Decaglyceryl monostearate (HLB = 12) | 25 g |
| Glycerin | 500 g |
| Pure water | 322 g |
| Sucrose stearate:<br>Ryoto Suger Ester S-1670 (HLB = 16) manufactured by Mitsubishi-Kagaku Foods Corporation<br>Decaglyceryl monostearate: NIKKOL Decaglyn 1-O (HLB =12) manufactured by Nikko Chemicals Co., Ltd. | |

Table 2

| Hematococcus alga extract (Astaxanthin content of 20 mass%) | 40 g |
|---|---|
| Mixed tocopherol | 10 g |
| Lecithin (derived from soy bean) | 90 g |
| Hematococcus alga extract: ASTOTS-S manufactured by Takeda Shiki Co., Ltd. Mixed tocopherol: RIKEN E oil 800 manufactured by Riken Vitamin Co. , Ltd. Lecithin (derived from soy bean): LECION P manufactured by Riken Vitamin Co., Ltd. | |

An aqueous composition (aqueous phase) was stirred at 10000 rpm by a homogenizer (Vacuum emulsifier PVQ-1D model, manufactured by MIZUHO Industrial CO,.LTD) while the temperature of the aqueous composition was maintained at 70°C, and the above-mentioned oil phase composition was added thereto, to thereby obtain an emulsion. The obtained emulsion was emulsified under a high pressure of 200 MPa at 40°C using Altimizer HJP-25005 (manufactured by Sugino Machine Limited).

Thereafter, the resultant emulsion was filtered through a microfilter having an average pore size of 1 $\mu$m to thereby prepare a clear-red astaxanthin-containing water dispersion (i). The obtained water dispersion had an average particle diameter of 100 nm or lower and had high transparency.

(2) Production of lotion bases (ii) and (iii)

Components 1 to 6 shown in Table 3 were mixed and dissolved at 80°C, and thereafter component 7 was added at 30°C to thereby obtain lotion bases (ii) and (iii).

Table 3

| No. | Formulation | Lotion base (ii) | Lotion base (iii) |
|---|---|---|---|
| 1 | Glycerin | 5.0 g | 4.0 g |
| 2 | 1,3-butylene glycol | 5.0 g | 4.0 g |
| 3 | Water | Balance | Balance |
| 4 | Methyl paraben | 0.2 g | 0.2 g |
| 5 | Xanthan gum | 0.45 g | - |
| 6 | Acetyl hydroxy proline | 0.1 g | 0.1 g |
| 7 | Magnesium ascorbyl phosphate | 1.0 g | 1.0 g |
| | Total | 100.0 g | 100.0 g |

(3) Production of lotion

To the lotion base (ii) or (iii), the water dispersion (i) and the respective other components were added and stirred at room temperature to thereby produce lotions.

* 1; Used as the water-soluble collagen was collagen which had an average molecular weight of 300,000 and which had been subjected to acid treatment.
*2; Used as the collagen peptide was hydrolysis collagen peptide having an average molecular weight of 3,000 and containing collagen tripeptide in a proportion of 10% or more.

Example 1

To 100 g of the lotion base (ii), 0.2 g of the water dispersion (i), 0.1 g of the water-soluble collagen (*1), and 0.1 g of the collagen peptide (*2) were added to thereby obtain a lotion a.

Example 2

To 100 g of the lotion base (iii), 0.05 g of the water dispersion (i), 0.1 g of the water-soluble collagen (*1), and 0.1 g of the collagen peptide (*2) were added to thereby obtain a lotion b.

Comparative Example 1

A lotion c was obtained in the same manner as in Example 1, except that the water dispersion (i) was not added.

Comparative Example 2

A lotion d was obtained in the same manner as in Example 1, except that the collagen peptide (*2) was not added.

Comparative Example 3

A lotion e was obtained in the same manner as in Example 1, except that the water-soluble collagen (*1) was not added.

Comparative Example 4

A lotion f was obtained in the same manner as in Example 1, except that the water dispersion (i) and the collagen peptide (*2) were not added.

Comparative Example 5

A lotion g was obtained in the same manner as in Example 1, except that the water dispersion (i) and the water-soluble collagen (*1) were not added.

Comparative Example 6

A lotion h was obtained in the same manner as in Example 1, except that the collagen peptide (*2) and the water-soluble collagen (*1) were not added.

(4) Production of cream base

The aqueous phase and the oil phase shown in Table 4 were emulsified at 80°C by a homomixer (Vacuum emulsifier PVQ-1D model, manufactured by MIZUHO Industrial CO,.LTD) to thereby obtain cream bases (iv) and (v).
Table 4

| | | Cream base (iv) | Cream base (v) |
|---|---|---|---|
| Aqueous phase | Water | Balance | Balance |
| | Glycerin | 7 | 7 |
| | 1,3-butylene glycol | 7 | 7 |
| | Methyl paraben | 0.2 | 0.2 |
| Oil phase | Surfactant | 3 | 3 |
| | Other oil-soluble component(s) | 20 | 20 |
| | Hematococcus alga extract (Astaxanthin content of 20 mass%) | 0.0055 | - |
| | Tocopherol | 0.5 | |

(continued)

|  | Cream base (iv) | Cream base (v) |
|---|---|---|
| Total | 100 | 100 |
| *The numbers in Table 4 each representing parts by weight | | |

(5) Production of cream

To the above-mentioned cream base (iv) or (v), other components were added and stirred at room temperature to thereby obtain a cream.

Example 3

To 100 g of the cream base (iv), 1 g of the water-soluble collagen (*1) and 0.1 g of the collagen peptide (*2) were added to thereby obtain a Cream i. The particle diameter of the cream was from 1 to 3 $\mu$m.

Comparative Example 7

A cream j was obtained in the same manner as in Example 3, except that the water-soluble collagen (*1) and the collagen peptide (*2) were not added. The particle diameter of the cream was from 1 to 3 $\mu$m.

Comparative Example 8

A cream k was obtained in the same manner as in Example 3, except that the cream base (v) was used in place of the cream base (iv). The particle diameter of the cream was from 1 to 3 $\mu$m.

[Evaluation of sense of use]

The sense of use of the lotions and creams obtained in Examples 1 to 3 and Comparative Examples 1 to 8 above was evaluated. Evaluation was performed by 10 in-house panel examiners (Women aged from 35 years old to 55). More specifically, each panel examiner sequentially used the lotions and creams obtained in Examples 1 to 3 and Comparative Examples 1 to 8 in the morning and evening for 1 week each, and evaluated, as anti-skin aging effects, the skin resiliency, the skin dullness, and the moist feeling of the skin sensed after using the respective lotions and creams based on the following criteria.

(1) Resiliency

3 points; Sensed increased resiliency
2 points; Sensed slightly increased resiliency
1 point; No change compared with the skin before use

(2) Skin dullness

3 points; Sensed improvement in skin dullness
2 points; Sensed slight improvement in skin dullness
1 point; No change compared with the skin before use

(3) Moist feeling

3 points; Sensed sufficient moist feeling
2 points; Sensed slight moist feeling

1 point; Sensed insufficient moist feeling

Table 5

| (Lotion) | | | | | | | | |
|---|---|---|---|---|---|---|---|---|
| Component | Example | | Comparative Example | | | | | |
| | Ex. 1 | Ex. 2 | Comp. Ex. 1 | Comp. Ex. 2 | Comp. Ex. 3 | Comp. Ex. 4 | Comp. Ex. 5 | Comp. Ex. 6 |
| | a | b | c | d | e | f | g | h |
| (A) Carotenoid | Added | Added | Not Added | Added | Added | Not Added | Not Added | Added |
| (B) Collagen Peptide | Added | Added | Added | Not Added | Added | Not Added | Added | Not Added |
| (C) Collagen | Added | Added | Added | Added | Not Added | Added | Not Added | Not Added |
| Evaluation | | | | | | | | |
| (1) Skin resiliency | 2.6 | 2.4 | 2.4 | 2.2 | 2.2 | 2.2 | 2 | 2 |
| (2) Skin dullness | 2.6 | 2.6 | 2.2 | 2.2 | 2.6 | 2 | 2.2 | 2.2 |
| (3) Moist feeling | 2.8 | 2.8 | 2.6 | 2.6 | 2.2 | 2.6 | 2 | 2.2 |

Table 6

| (Cream) | | | |
|---|---|---|---|
| Component | Ex. 3 | Comp. Ex. 7 | Comp. Ex. 8 |
| | i | j | k |
| (A) Carotenoid | Added | Added | Not Added |
| (B) Collagen Peptide | Added | Not Added | Added |
| (C) Collagen | Added | Not Added | Added |
| Evaluation | | | |
| (1) Skin resiliency | 2.6 | 2.4 | 2.2 |
| (2) Skin dullness | 2.6 | 2.2 | 2.2 |
| (3) Moist feeling | 2.6 | 2.2 | 2.8 |

As is clear from Tables 5 and 6, it was found that the sense of use with improved skin resiliency, decreased skin dullness, and enhanced moist feeling was obtained in all of the Examples, as compared with the Comparative Examples. More specifically, the Examples demonstrated outstanding anti-skin aging effects as compared with the Comparative Examples.

[Skin elasticity evaluation]

Using the lotion a of Example 1, the skin elasticity was evaluated based on the changes in the skin elasticity. The skin elasticity of each of 5 subjects (women aged from 35 to 55) was measured with CUTOMETERMPA580 (manufactured by Integral Corporation).
Measurement was performed twice before and after use (two-week use of the lotion in the morning and evening). The measurement results are shown in Table 7.

Table 7

| <Skin elasticity> | | |
|---|---|---|
| Use Period | 0 day | 14 days |
| Skin elasticity (Average recovery ratio $\pm$ SD) | 63.1 $\pm$ 3.3 | 81.8 $\pm$ 3.7 |
| | | (unit: %) |

As is clear from Table 7, it was found that the skin elasticity significantly increased by the use of the lotion a of Example 1.

**Claims**

1. A cosmetic composition, comprising:

   a carotenoid (A),
   a collagen (B) having a weight average molecular weight of 40,000 or more, and
   a collagen peptide (C) having a weight average molecular weight of from 200 to 5,000.

2. The cosmetic composition according to claim 1, wherein the carotenoid (A) is at least one selected from the group consisting of astaxanthin and derivatives thereof.

3. The cosmetic composition according to claim 1, further comprising at least one selected from the group consisting of ascorbic acid and derivatives thereof, and tocopherol and derivatives thereof.

4. The cosmetic composition according to claim 3, wherein the at least one selected from the group consisting of ascorbic acid and derivatives thereof, and tocopherol and derivatives thereof includes at least one selected from the group consisting of magnesium ascorbyl phosphorate, sodium ascorbyl phosphate, ascorbyl-2-glucoside, and sodium ascorbate.

5. The cosmetic composition according to claim 3, wherein the at least one selected from the group consisting of ascorbic acid and derivatives thereof, and tocopherol and derivatives thereof includes at least one member selected from the group consisting of tocopherol and tocotrienol.

6. The cosmetic composition according to claim 1, wherein the collagen peptide (C) includes an oligopeptide having 2 to 6 peptide bonds.

7. The cosmetic composition according to claim 1, further comprising an amino acid or a derivative thereof.

8. The cosmetic composition according to claim 1, wherein the collagen peptide (C) is derived from a fish.

9. The cosmetic composition according to claim 1, wherein the collagen (B) is derived from a fish.

10. The cosmetic composition according to claim 1, wherein the carotenoid (A) is contained in emulsion particles in an oil-in-water water dispersion.

11. The cosmetic composition according to claim 10, wherein the volume average particle diameter of the emulsion particles is 200 nm or less.

**Patentansprüche**

1. Kosmetikzusammensetzung, die Folgendes umfasst:

   ein Carotinoid (A),
   ein Collagen (B) mit einem gewichtsmittleren Molekulargewicht von 40 000 oder mehr sowie
   ein Collagenpeptid (C) mit einem gewichtsmittleren Molekulargewicht von 200 bis 5 000.

2. Kosmetikzusammensetzung nach Anspruch 1, wobei es sich bei dem Carotinoid (A) um mindestens eines, das aus der Gruppe bestehend aus Astaxanthin und seinen Derivaten ausgewählt ist, handelt.

3. Kosmetikzusammensetzung nach Anspruch 1, die weiterhin mindestens eine Substanz, die aus der Gruppe bestehend aus Ascorbinsäure und ihren Derivaten sowie Tocopherol und seinen Derivaten ausgewählt ist, umfasst.

4. Kosmetikzusammensetzung nach Anspruch 3, wobei die mindestens eine aus der Gruppe bestehend aus Ascorbinsäure und ihren Derivaten und Tocopherol und seinen Derivaten ausgewählte Substanz mindestens eine aus der Gruppe bestehend aus Magnesiumascorbylphosphat, Natriumascorbylphosphat, Ascorbyl-2-Glucosid und Natriumascorbat ausgewählte Substanz beinhaltet.

5. Kosmetikzusammensetzung nach Anspruch 3, wobei die mindestens eine aus der Gruppe bestehend aus Ascorbinsäure und ihren Derivaten und Tocopherol und seinen Derivaten ausgewählte Substanz mindestens ein Mitglied, das aus der Gruppe bestehend aus Tocopherol und Tocotrienol ausgewählt ist, beinhaltet.

6. Kosmetikzusammensetzung nach Anspruch 1, wobei das Collagenpeptid (C) ein Oligopeptid mit 2 bis 6 Peptidbindungen beinhaltet.

7. Kosmetikzusammensetzung nach Anspruch 1, die weiterhin eine Aminosäure oder ein Derivat davon umfasst.

8. Kosmetikzusammensetzung nach Anspruch 1, wobei das Collagenpeptid (C) von einem Fisch stammt.

9. Kosmetikzusammensetzung nach Anspruch 1, wobei das Collagen (B) von einem Fisch stammt.

10. Kosmetikzusammensetzung nach Anspruch 1, wobei das Carotinoid (A) in Emulsionspartikeln in einer Öl-in-Wasser-Wasserdispersion enthalten ist.

11. Kosmetikzusammensetzung nach Anspruch 10, wobei der volumenmittlere Teilchendurchmesser der Emulsionsteilchen 200 nm oder weniger beträgt.

**Revendications**

1. Composition cosmétique, comprenant :

   un caroténoïde (A),
   un collagène (B) ayant un poids moléculaire moyen en poids de 40 000 ou plus, et
   un peptide de collagène (C) ayant un poids moléculaire moyen en poids de 200 à 5 000.

2. Composition cosmétique selon la revendication 1, dans laquelle le caroténoïde (A) est au moins l'un choisi dans le groupe constitué de l'astaxanthine et de dérivés de celle-ci.

3. Composition cosmétique selon la revendication 1, comprenant en outre au moins l'un choisi dans le groupe constitué de l'acide ascorbique et de dérivés de celui-ci, et du tocophérol et de dérivés de celui-ci.

4. Composition cosmétique selon la revendication 3, dans laquelle l'au moins un choisi dans le groupe constitué de l'acide ascorbique et de dérivés de celui-ci ; et du tocophérol et de dérivés de celui-ci comprend au moins l'un choisi dans le groupe constitué de l'ascorbyl-phosphorate de magnésium, l'ascorbyl-phosphate de sodium, l'ascorbyl-2-glucoside, et l'ascorbate de sodium.

**5.** Composition cosmétique selon la revendication 3, dans laquelle l'au moins un choisi dans le groupe constitué de l'acide ascorbique et de dérivés de celui-ci, et du tocophérol et de dérivés de celui-ci comprend au moins un membre choisi dans le groupe constitué du tocophérol et du tocotriénol.

**6.** Composition cosmétique selon la revendication 1, dans laquelle le peptide de collagène (C) comprend un oligopeptide ayant de 2 à 6 liaisons peptidiques.

**7.** Composition cosmétique selon la revendication 1, comprenant en outre un acide aminé ou un dérivé de celui-ci.

**8.** Composition cosmétique selon la revendication 1, dans laquelle le peptide de collagène (C) est dérivé d'un poisson.

**9.** Composition cosmétique selon la revendication 1, dans laquelle le collagène (B) est dérivé d'un poisson.

**10.** Composition cosmétique selon la revendication 1, dans laquelle le caroténoïde (A) est contenu dans des particules d'émulsion dans une dispersion aqueuse d'huile dans l'eau.

**11.** Composition cosmétique selon la revendication 10, dans laquelle le diamètre de particule moyen en volume des particules d'émulsion est de 200 nm ou moins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2049091 A
- JP 5155736 A
- JP 2005047860 A
- JP 2004244369 A
- JP 2006151847 A
- JP 2006342107 A
- JP 8103288 A
- JP 5068585 A
- JP 7082299 A

**Non-patent literature cited in the description**

- **DAVIES, B.H.** Chemistry and Biochemistry of Plant Pigments. Academic Press, 1976, 38-165
- **YAMAGUCHI, K. ; MIKI, W. ; TORIU, N. ; KONDO, Y. ; MURAKAMI, M. ; KONOSU, S. ; SATAKE, M. ; FUJITA, T.** The composition of carotenoid pigments in the antarctic krill Euphausia superba. *Bull. Jap. Sos. Sci. Fish.,* 1983, vol. 49, 1411-1415
- **RENSTROM, B. ; LIAAEA-JENSEN, S.** Fatty acids of some esterified carotenols. *Comp. Biochem. Physiol. B, Comp. Biochem.,* 1981, vol. 69, 625-627
- **ANDREWES, A.G. ; STARR, M.P.** (3R, 3'R)-Asttaxanthin from the yeast Phaffa rhodozyma. *Phytochem.,* 1976, vol. 15, 1009-1011
- **ANDREWES, A.G. ; PHAFFIA, HJ. ; STARR, M.P.** Carotenids of Phaffia rhodozyma, a red pigmented fermenting yeast. *Phytochem.,* 1976, vol. 15, 1003-1007
- **KUHN, R. ; SOERENSEN, N.A.** The coloring matters of the lobster (Astacus gammarusL.). *Z. Angew. Chem.,* 1938, vol. 51, 465-466
- **CHEESMAN, D.F.** Ovorubin, a chromoprotein from the eggs of the gastropod mollusc Pomacea canaliculata. *Proc. Roy. Soc.B,* 1958, vol. 149, 571-587